# EUROPÄISCHE PATENTSCHRIFT

(11) **EP 1 197 472 B1**
(45) Veröffentlichungstag und Bekanntmachung des Hinweises auf die Patenterteilung: **19.01.2011**
(21) Anmeldenummer: 00120893.3
(22) Anmeldetag: 26.09.2000
(51) Int. Cl.: C01B 13/22

(54) **Eisenoxid- und Siliciumdioxid-Titandioxid-Mischung**
Iron oxide- and silicon dioxide- titanium dioxide mixture
Mélange d' oxyde de fer de dioxyde de silicium et de dioxyde de titane

(43) Veröffentlichungstag der Anmeldung: 17.04.2002
(73) Patentinhaber: Evonik Degussa GmbH, 45128 Essen (DE)
(72) Erfinder: Hemme, Ina, Dr., 63450 Hanau (DE); Habermann, Herbert, 63599 Biebergemünd (DE); Hasenzahl, Steffen, Dr., 63477 Maintal (DE)

(56) Entgegenhaltungen:
- EP-A- 0 595 078
- EP-A- 0 609 533
- US-A- 4 297 143
- US-A- 6 022 404

## Beschreibung

Die Erfindung betrifft ein Eisenoxid-Siliciumdioxid-Titandioxid-Mischoxid, das Verfahren zu seiner Herstellung und seine Verwendung.

Es ist bekannt, Titandioxid auf pyrogenem Wege, vor allem mittels des flammenhydrolytischen Verfahrens herzustellen (DE-PS 830 786). Ein derartig hergestelltes Titandioxid kann in Sonnenschutzmitteln als UV-absorbierendes Mittel bei gleichzeitiger Transparenz für sichtbares Licht eingesetzt werden. Eine weitere Einsatzmöglichkeit besteht in Lacken oder Kunststoffen.

Die Herstellung von Mischoxiden auf pyrogenem Wege ist bekannt aus den Dokumenten DE-A 952 891, DE-A 29 31 585, DE-A 24 31 810 und DE-A 36 11 449.

Die JP-A-5330825 beschreibt mit Eisenoxid dotiertes Titandioxid, das auf dem Fällungswege hergestellt wird und vorzugsweise mit weiteren Oxiden beschichtet ist.

Mittels Flammenhydrolyse kann, wie im Patent EP 0 609 533 B1 beschrieben, auch eisenoxidhaltiges Titandioxidpulver hergestellt werden. Für besondere Einsatzzwecke, wie zum Beispiel als UV-absorbierendes Agens in Sonnenschutzmitteln, ist eine besonders hohe Absorptionsfähigkeit für UV-Strahlung insbesondere für UVB-Strahlung und eine Transparenz, die mit dem bekannten Titandioxid nicht erreicht werden kann, notwendig.

Gegenstand der Erfindung ist ein Eisenoxid-Siliciumdioxid-Titandioxid-Mischoxid, hergestellt auf pyrogenem Wege, insbesondere auf flammenhydrolytischen Wege. Das kann aus einem pyrogen, insbesondere flammenhydrolytisch hergestellten, ternären Eisenoxid-Siliciumdioxid-Titandioxid-Mischoxid mit einer spezifischen Oberfläche von 10 - 200 m²/g, das 0,5 bis 50 Gew.-% Eisenoxid und 0,5 - 50 Gew.-% Siliciumdioxid, bezogen auf die Gesamtmenge, als Bestandteile des ternären Mischoxids enthält, bestehen.

Ein weiterer Gegenstand der Erfindung ist ein Verfahren zur Herstellung des Eisenoxid-Siliciumdioxid-Titandioxid-Mischoxides, welches dadurch gekennzeichnet ist, dass man verdampfbare Eisenverbindungen verdampft und in die Mischkammer eines bekannten Brenners überführt, gleichzeitig verdampfbare Siliciumverbindungen und Titanverbindungen separat dosiert und gemeinsam verdampft und ebenfalls in die Mischkammer des bekannten Brenners überführt, wobei die verdampften gasförmigen Verbindungen in einem Verhältnis, das der Zusammensetzung des Eisenoxid-Siliciumdioxid-Titandioxid-Mischoxids entspricht, dosiert werden, die gasförmigen Verbindungen in der Mischkammer des bekannten Brenners mit (Kern-)Wasserstoff und (Kern-)Sauerstoff und/oder (Kern-)Luft vermischt, und das Gasgemisch, das aus der Düsenöffnung des Brenners strömt, in der Reaktionskammer des Brenners verbrennt, wobei zusätzlich (Mantel-)Wasserstoff und gegebenenfalls (Mantel-)Stickstoff in die den Brennermund umgebende Ringdüse eingespeist werden, das entstandene Eisenoxid-Siliciumdioxid-Titandioxid-Mischoxid von den gasförmigen Reaktionsprodukten abtrennt und gegebenenfalls mittels Wasserdampf von anhaftenden Reaktionsprodukten befreit.

Das erfindungsgemässe Eisenoxid-Siliciumdioxid-Titandioxid-Mischoxid kann hergestellt werden, indem man wasserfreies Eisen (III)-chlorid verdampft, zusammen mit einem Inertgas, zum Beispiel Stickstoff, in die Mischkammer eines bekannten Brenners überführt, dort mit Wasserstoff, Luft und einem getrennt dosierten, aber gemeinsam verdampften Gemisch aus gasförmigem Titantetrachlorid und Siliciumtetrachlorid, gegebenenfalls mit einem Trägergas, das inert sein kann, wie zum Beispiel Stickstoff und/oder Luft, in einem Verhältnis, das der Zusammensetzung des Eisenoxid-Siliciumdioxid-Titandioxid-Mischoxids entspricht, vermischt, das Mehrkomponentengemisch in die Reaktionskammer (Flammenrohr) des Brenners verbrennt, wobei zusätzlich (Mantel-)Wasserstoff und gegebenenfalls (Mantel-)Stickstoff in die den Brennermund umgebende Ringdüse eingespeist werden, danach das feste von den gasförmigen Reaktionsprodukten abtrennt und gegebenenfalls in feuchter Luft von anhaftenden Reaktionsprodukten befreit.

Bei dem erfindungsgemässen Verfahren kann eine Brennervorrichtung verwendet werden, wie sie in dem Dokument EP 0 814 057 B1 beschrieben wird.

In einer bevorzugten Ausführungsform kann das Eisenoxid-Siliciumdioxid-Titandioxid-Mischoxid die folgenden physikalisch-chemischen Daten aufweisen:

| | |
|---|---|
| Titandioxid-Gehalt (Gew.-%) | 0,5 - 99,0 |
| Eisenoxid-Gehalt (Gew.-%) | 0,5 - 50 |
| Siliciumdioxid-Gehalt (Gew.-%) | 0,5 - 50 |
| Spezifische Oberfläche (m²/g) | 10 - 200 |
| Primärteilchengröße (nm) | 5 - 120 |
| Stampfdichte (g/l) | 100 - 400 |
| Glühverlust (2h, 1000°C) (Gew.-%) | 0,5 - 5 |
| Chloridgehalt (Gew.-%) | < 1 |

Das erhaltene das Eisenoxid-Siliciumdioxid-Titandioxid-Mischoxid ist sehr feinteilig, sehr homogen und sehr rein. Es weist gegenüber dem Stand der Technik eine bessere Absorption und damit eine schlechtere Transparenz von UV-Licht bei einer weitgehenden Transparenz für sichtbares Licht auf. Es ist gut im entsprechenden Medium, zum Beispiel einem Sonnenschutzmittel, dispergierbar.

Das Eisenoxid-Siliciumdioxid-Titandioxid-Mischoxid kann weiterhin zur Herstellung von kosmetischen Artikeln, Lacken, Katalysatoren, Katalysatorträgern und Photokatalysatoren und als UV-Absorber eingesetzt werden.

Ein weiterer Gegenstand der Erfindung sind Hautkosmetika, die das erfindungsgemässe Eisenoxid-Siliciumdioxid-Titandioxid-Mischoxid vorzugsweise in einer Menge von 0,05 - 10 Gew.-%, enthalten.

### Beispiel 1

FeCl₃, SiCl₄ und TiCl₄ werden in drei getrennten Dosiereinrichtungen eindosiert und in zwei getrennten Verdampfern verflüchtigt (Verdampfertemperaturen für FeCl₃ 350°C und für TiCl₄ und SiCl₄ 200°C). Dabei werden SiCl₄ und TiCl₄ getrennt dosiert aber gemeinsam in einem Verdampfer verflüchtigt. FeCl₃ wird ebenfalls getrennt dosiert, aber separat verflüchtigt. Die Chloriddämpfe werden mittels Stickstoff in die Mischkammer des Brenners geleitet. Dort werden sie mit (Kern-)Wasserstoff und getrockneter (Kern-)Luft und/oder (Kern-)Sauerstoff vermischt und in eine Reaktionskammer (Flammenrohr) verbrannt zusätzlich wird (Mantel-)Wasserstoff und gegebenenfalls (Mantel-)Stickstoff in die den Brennermund umgebende Ringdüse eingespeist. In der Koagulationsstrecke wird das Eisenoxid-Siliciumdioxid-Titandioxid-Mischoxid auf ca. 100°C abgekühlt. Das erhaltene Eisenoxid-Siliciumdioxid-Titandioxid-Mischoxid wird anschließend mit einem Filter abgeschieden. Durch die Behandlung des Eisenoxid-Siliciumdioxid-Titandioxid-Mischoxid mit feuchter Luft bei Temperaturen zwischen 500°C und 700°C wird anhaftendes Chlorid entfernt.

In der Tabelle 1 sind die Reaktionsbedingungen für die Herstellung des Eisenoxid-Siliciumdioxid-Titandioxid-Mischoxid zusammengestellt.

Tabelle 2 zeigt die Produkteigenschaften des Eisenoxid-Siliciumdioxid-Titandioxid-Mischoxids.

**Tabelle 1: Experimentelle Bedingungen bei der Herstellung des ternären pyrogenen Eisenoxid-Siliciumdioxid-Titandioxid-Mischoxids**

| Beispiel | TiCl₄ | SiCl₄ | FeCl₃ | H₂ Kern | H₂ Mantel | N₂ Kern | N₂ Mantel | Primärluft | O₂ Kern | Gas-Temp. |
|---|---|---|---|---|---|---|---|---|---|---|
| Nr. | [kg/h] | [kg/h] | [kg/h] | [m³ /h] | [m³ /h] | [m³ /h] | [m³ /h] | [m³ /h] | [m³ /h] | [°C] |
| 1 | 1,31 | 0,37 | 0,02 | 0,42 | 0,10 | 0,54 | 0,13 | 2,78 | 0,13 | 350 |

| | | | | | | | | | | |
|---|---|---|---|---|---|---|---|---|---|---|
| Erläuterung: Primärluft = Luftmenge im Zentralrohr; H₂ Kern = Wasserstoff im Zentralrohr; H₂ Mantel = Mantelwasserstoff; N₂ Kern = Stickstoff im Zentralrohr; ; N₂ Mantel = Mantelstickstoff; ; O₂ Kern = Sauerstoff im Zentralrohr; Gas-Temp. = Gastemperatur in der Düse des Zentralrohres | | | | | | | | | | |

**Tabelle 2**

| Beispiel Nr. | TiO₂ (%) | SiO₂ (%) | Fe₂O₃ (%) | pH (4%ige wäßrige Suspension) | Spez. Oberfläche (BET) (m²/g) | Stampfdichte (g/l) | Glühverlust (%) | Chloridgehalt (%) |
|---|---|---|---|---|---|---|---|---|
| 1 | 80,07 | 18,51 | 1,35 | 3,89 | 85 | 82 | 1,07 | 0,07 |

| | | | | | | | | |
|---|---|---|---|---|---|---|---|---|
| Glühverlust(2 h bei 1000°C, in Anlehnung an DIN 55921, ASTM D 1208, JIS K 5101, bezogen auf die 2 h bei 105°C getrocknete Substanz); pH = pH-Wert in 4%iger wässriger Suspension; Stampfdichte in Anlehnung an DIN/ISO 787/XI, JISK 5101/18 (nicht gesiebt) | | | | | | | | |

### Messung der UV-Absorption

Zur Messung der UV-Absorption beziehungsweise der Transmission werden 3% des Eisenoxid-Siliciumdioxid-Titandioxid-Mischoxids gemäss Beispiel 1 mit der Zusammensetzung 1,35 Gew.-% Fe₂O₃, 18,51 Gew.-% SiO₂, 80,07 Gew.-% TiO₂ in einer Mischung mit 8 Gew.-% Aerosil 200 und 89 Gew.-% Isopropylpalmitat dispergiert. Anschließend wird die Dispersion in eine Quarzküvette mit einer Schichtdicke von 10µm gefüllt. Mit einem Spektrometer UV-201 der Firma Shimazu wird die Transmission gemessen. Als Vergleichssubstanz wird das pyrogene Titandioxid P 25 (Spez. Oberfläche 50 ± 15 m²/g) verwendet und unter den gleichen Bedingungen untersucht.

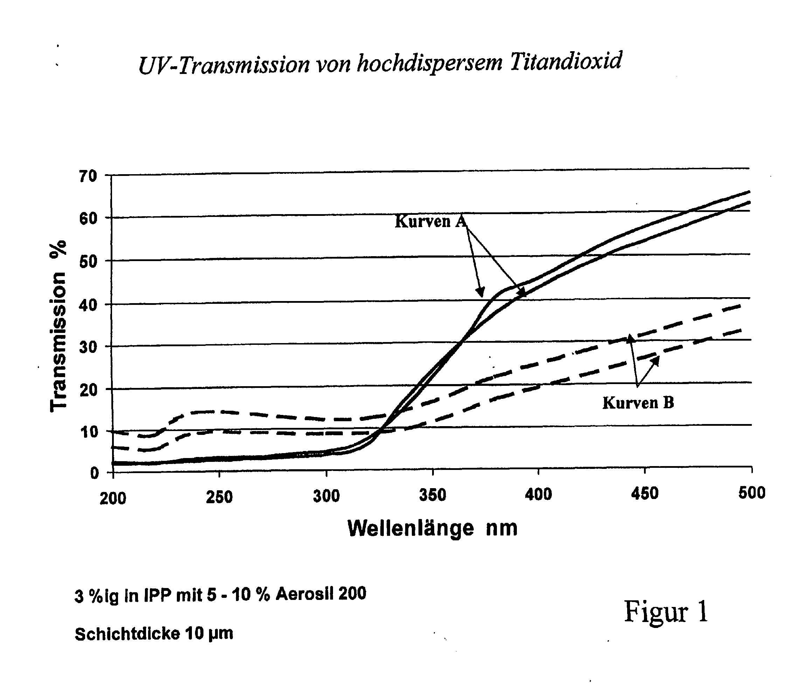

Figur 1 zeigt die Abhängigkeit der Transmission in % von der Wellenlänge im Bereich von 200 - 400nm (Kurve A: erfindungsgemäßes Eisenoxid-Siliciumdioxid-Titandioxid-Mischoxid, Kurve B: Titandioxid P 25). Die Dispersion mit dem erfindungsgemässen Mischoxid zeigt gegenüber der Dispersion, die reines Titandioxid P 25 enthält, eine deutlich geringere Transmission im UVB-Bereich. Das erfindungsgemässe Eisenoxid-Siliciumdioxid-Titandioxid-Mischoxid bietet damit einen ausgezeichneten Schutz gegen UV-Strahlung, insbesondere im besonders schädlichen UVB-Bereich.

## Patentansprüche

1. Eisenoxid-Siliciumdioxid-Titandioxid-Mischoxid, hergestellt auf pyrogenem Wege.

2. Eisenoxid-Siliciumdioxid-Titandioxid-Mischoxid gemäß Anspruch 1, **dadurch gekennzeichnet, dass** es auf flammenhydrolytischem Wege hergestellt ist.

3. Eisenoxid-Siliciumdioxid-Titandioxid-Mischoxid nach den Ansprüchen 1 oder 2, **dadurch gekennzeichnet, dass** es aus einem pyrogen hergestellten Eisenoxid-Siliciumdioxid-Titandioxid-Mischoxid mit einer spezifischen Oberfläche von 10 bis 200 m²/g, welches 0,5 bis 50 Gew.-%. Eisenoxid und 0,5 - 50% Siliciumdioxid, bezogen auf die Gesamtmenge, als Bestandteil des Mischoxids enthält, besteht.

4. Verfahren zur Herstellung des Eisenoxid-Siliciumdioxid-Titandioxid-Mischoxides gemäß Anspruch 1 bis 3, **dadurch gekennzeichnet, dass** man verdampfbare Eisenverbindungen verdampft und in die Mischkammer eines bekannten Brenners überführt, gleichzeitig verdampfbare Siliciumverbindungen und Titanverbindungen separat dosiert und gemeinsam verdampft und ebenfalls in die Mischkammer des bekannten Brenners überführt, wobei die verdampften gasförmigen Verbindungen in einem Verhältnis, das der Zusammensetzung des Eisenoxid-Siliciumdioxid-Titandioxid-Mischoxides entspricht, dosiert werden, die gasförmigen Verbindungen in der Mischkammer des bekannten Brenners mit (Kern-)Wasserstoff und (Kern-)Sauerstoff und/oder (Kern-)Luft vermischt und das Gasgemisch in die Reaktionskammer des Brenners verbrennt wobei zusätzlich (Mantel-)Wasserstoff und gegebenenfalls (Mantel-)Stickstoff in die den Brennermund umgebende Ringdüse eingespeist werden, und das entstandene Eisenoxid-Siliciumdioxid-Titandioxid-Mischoxid von den gasförmigen Reaktionsprodukten abtrennt und gegebenenfalls mittels Wasserdampf von anhaftenden Reaktionsprodukten befreit.

5. Verfahren zur Herstellung des Eisenoxid-Siliciumdioxid-Titandioxid-Mischoxides nach Anspruch 4, **dadurch gekennzeichnet, dass** man wasserfreies Eisen (III)-chlorid verdampft, zusammen mit einem Inertgas in die Mischkammer eines bekannten Brenners überführt, dort mit Wasserstoff, Luft und einem getrennt dosierten, aber gemeinsam verdampften Gemisch aus gasförmigem Titantetrachlorid und Siliciumtetrachlorid, gegebenenfalls mit einem Traggas, das inert sein kann wie zum Beispiel Stickstoff, und/oder Luft in einem Verhältnis, das der Zusammensetzung des Eisenoxid-Siliciumdioxid-Titandioxid-Mischoxides entspricht, vermischt, zusätzlich (Mantel-)Wasserstoff und gegebenenfalls (Mantel-)Stickstoff in die den Brennermund umgebende Ringdüse einspeist, das Mehrkomponentengemisch in der Reaktionskammer des Brenners verbrennt, danach das feste Eisenoxid-Siliciumdioxid-Titandioxid-Mischoxid von den gasförmigen Reaktionsprodukten abtrennt und gegebenenfalls in feuchter Luft von anhaftenden Reaktionsprodukten befreit.

6. Verwendung des pyrogen hergestellten Eisenoxid-Siliciumdioxid-Titandioxid-Mischoxids nach Anspruch 1 oder 2 zur Herstellung von kosmetischen Artikeln, Lacken, Katalysatoren, Katalysatorträgern, Photokatalysatoren und als UV-Absorber.

7. Hautkosmetika, enthaltend Eisenoxid-Siliciumdioxid-Titandioxid-Mischoxid gemäß Anspruch 1 oder 2, vorzugsweise in einer Menge von 0,05 bis 10 Gew.-%.

## Claims

1. Iron oxide-silicon dioxide-titanium dioxide mixed oxide produced pyrogenically.

2. Iron oxide-silicon dioxide-titanium dioxide mixed oxide according to Claim 1, **characterized in that** it is produced flame-hydrolytically.

3. Iron oxide-silicon dioxide-titanium dioxide mixed oxide according to Claim 1 or 2, **characterized in that** it consists of a pyrogenically produced iron oxide-silicon dioxide-titanium dioxide mixed oxide having a specific surface area of 10 to 200 m²/g and containing 0.5 to 50 wt.% iron oxide and 0.5-50% silicon dioxide, based on the total amount, as constituent of the mixed oxide.

4. Process for producing the iron oxide-silicon dioxide-titanium dioxide mixed oxide according to any of Claims 1 to 3, **characterized in that** vaporizable iron compounds are vaporized and transferred into the mixing chamber of a known burner while at the same time vaporizable silicon compounds and titanium compounds are separately metered and conjointly vaporized and likewise transferred into the mixing chamber of the known burner, wherein the vaporized gaseous compounds are metered in a ratio which corresponds to the composition of the iron oxide-silicon dioxide-titanium dioxide mixed oxide, the gaseous compounds are mixed with (core) hydrogen and (core) oxygen and/or (core) air in the mixing chamber of the known burner and the gas mixture is burned in the reaction chamber of the burner, wherein additionally (shell) hydrogen and optionally (shell) nitrogen are fed into the ring nozzle surrounding the burner throat, and the resulting iron oxide-silicon dioxide-titanium dioxide mixed oxide is separated from the gaseous reaction products and optionally freed of adhering reaction products using water vapour.

5. Process for producing the iron oxide-silicon dioxide-titanium dioxide mixed oxide according to Claim 4, **characterized in that** anhydrous iron(III) chloride is vaporized, transferred together with an inert gas into the mixing chamber of a known burner, mixed therein with hydrogen, air and a separately metered but conjointly vaporized mixture of gaseous titanium tetrachloride and silicon tetrachloride, optionally with a carrier gas, which can be inert such as for example nitrogen, and/or air in a ratio which corresponds to the composition of the iron oxide-silicon dioxide-titanium dioxide mixed oxide, (shell) hydrogen and optionally (shell) nitrogen is additionally fed into the ring nozzle surrounding the burner throat, the multicomponent mixture is burned in the reaction chamber of the burner, thereafter the solid iron oxide-silicon dioxide-titanium dioxide mixed oxide is separated from the gaseous reaction products and optionally freed of adhering reaction products in moist air.

6. Use of the pyrogenically produced iron oxide-silicon dioxide-titanium dioxide mixed oxide according to Claim 1 or 2 in the manufacture of cosmetic articles, coatings, catalysts, catalyst carriers, photocatalysts and as UV absorber.

7. Skin cosmetics containing iron oxide-silicon dioxide-titanium dioxide mixed oxide according to Claim 1 or 2, preferably in an amount of 0.05 to 10 wt.%.

## Revendications

1. Oxyde mixte d'oxyde de fer-dioxyde de silicium-dioxyde de titane, préparé par voie pyrogène.

2. Oxyde mixte d'oxyde de fer-dioxyde de silicium-dioxyde de titane selon la revendication 1, **caractérisé en ce qu'**il est préparé par voie hydrolytique à la flamme.

3. Oxyde mixte d'oxyde de fer-dioxyde de silicium-dioxyde de titane selon les revendications 1 ou 2, **caractérisé en ce qu'**il est constitué par un oxyde mixte d'oxyde de fer-dioxyde de silicium-dioxyde de titane préparé par voie pyrogène présentant une surface spécifique de 10 à 200 m²/g, qui contient 0,5 à 50% en poids d'oxyde de fer et 0,5-50% de dioxyde de silicium, par rapport à la quantité totale, comme constituant de l'oxyde mixte.

4. Procédé pour la préparation de l'oxyde mixte d'oxyde de fer-dioxyde de silicium-dioxyde de titane selon la revendication 1 à 3, **caractérisé en ce qu'**on évapore et on transfère dans la chambre de mélange d'un brûleur connu des composés du fer pouvant être évaporés, on dose séparément et on évapore simultanément des composés du silicium et du titane pouvant être évaporés simultanément et on les transfère également dans la chambre de mélange du brûleur connu, les composés gazeux évaporés étant dosés dans un rapport qui correspond à la composition de l'oxyde mixte d'oxyde de fer-dioxyde de silicium-dioxyde de titane, on mélange les composés gazeux dans la chambre de mélange du brûleur connu avec de l'hydrogène (noyau) et de l'oxyde (noyau) et/ou de l'air (noyau) et on brûle le mélange gazeux dans la chambre de réaction du brûleur, en injectant en outre de l'hydrogène (enveloppe) et le cas échéant de l'azote (enveloppe) dans le gicleur annulaire entourant l'orifice du brûleur et on sépare l'oxyde mixte d'oxyde de fer-dioxyde de silicium-dioxyde de titane formé des produits de réaction gazeux et on le libère le cas échéant des produits de réaction qui adhèrent au moyen de vapeur d'eau.

5. Procédé pour la préparation de l'oxyde mixte d'oxyde de fer-dioxyde de silicium-dioxyde de titane selon la revendication 4, **caractérisé en ce qu'**on évapore du chlorure de fer (III) anhydre, on le transfère ensemble avec un gaz inerte dans la chambre de mélange d'un brûleur connu, on l'y mélange avec de l'hydrogène, de l'air et un mélange dosé séparément, mais évaporé ensemble, de tétrachlorure de titane et de tétrachlorure de silicium gazeux, le cas échéant avec un gaz support, qui peut être inerte, tel que par exemple l'azote, et/ou de l'air dans un rapport qui correspond à la composition de l'oxyde mixte d'oxyde de fer-dioxyde de silicium-dioxyde de titane, on injecte en outre de l'hydrogène (enveloppe) et le cas échéant de l'azote (enveloppe) dans le gicleur annulaire entourant l'orifice du brûleur, on brûle le mélange à plusieurs composants dans la chambre de réaction du brûleur, on sépare ensuite l'oxyde mixte d'oxyde de fer-dioxyde de silicium-dioxyde de titane solide des produits de réaction gazeux et on le libère le cas échéant dans l'air humide des produits de réaction qui adhèrent.

6. Utilisation de l'oxyde mixte d'oxyde de fer-dioxyde de silicium-dioxyde de titane selon la revendication 1 ou 2 pour la préparation d'articles cosmétiques, de laques, de catalyseurs, de supports de catalyseur, de photocatalyseurs et d'absorbants des UV.

7. Cosmétiques pour la peau, contenant de l'oxyde mixte d'oxyde de fer-dioxyde de silicium-dioxyde de titane selon la revendication 1 ou 2, de préférence en une quantité de 0,05 à 10% en poids.
